# EUROPÄISCHE PATENTSCHRIFT

(11) **EP 3 505 216 B1**
(45) Veröffentlichungstag und Bekanntmachung des Hinweises auf die Patenterteilung: **22.07.2020**
(21) Anmeldenummer: 18150083.6
(22) Anmeldetag: 02.01.2018
(51) Int. Cl.: A61N 1/375

(54) **ELEKTRISCHE KONTAKTIERUNGSVORRICHTUNGSVORRICHTUNG FÜR EINE IMPLANTIERBARE MEDIZINISCHE VORRICHTUNG UND VERFAHREN ZUR HERSTELLUNG**
ELECTRICAL CONTACTING DEVICE FOR AN IMPLANTABLE MEDICAL DEVICE AND METHOD FOR PRODUCING
DISPOSITIF DE MISE EN CONTACT ÉLECTRIQUE POUR UN DISPOSITIF MÉDICAL IMPLANTABLE ET PROCÉDÉ DE FABRICATION

(43) Veröffentlichungstag der Anmeldung: 03.07.2019
(73) Patentinhaber: Heraeus Deutschland GmbH & Co. KG, 63450 Hanau (DE)
(72) Erfinder: Trötzschel, Jens, 63549 Ronneburg (DE); Nikolaidis, Ilias, 60598 Frankfurt am Main (DE); Dittmer, Robert, 63450 Hanau (DE); Hausch, Ulrich, 60318 Frankfurt am Main (DE)
(74) Vertreter: Heraeus IP

(56) Entgegenhaltungen:
- EP-A1- 1 934 994
- EP-A1- 2 837 446
- US-A1- 2011 190 885
- US-A1- 2012 197 326
- US-A1- 2012 200 011

## Beschreibung

Die Erfindung betrifft eine elektrische Kontaktierungsvorrichtung für eine medizinische implantierbare Vorrichtung; eine medizinische implantierbare Vorrichtung, aufweisend eine Kontaktierungsvorrichtung; ein Verfahren zur Herstellung einer elektrischen Kontaktierungsvorrichtung; und die Verwendung einer elektrischen Kontaktierungsvorrichtung.

### STAND DER TECHNIK

Die DE 10 2011 009 858 B4 beschreibt eine elektrische Durchführung zum Einsatz in einem Gehäuse einer medizinisch implantierbaren Vorrichtung. Die elektrische Durchführung verfügt über einen elektrisch isolierenden Grundkörper aus Keramik und mindestens ein elektrisches Leitungselement. Das Leitungselement ist eingerichtet, um durch den Grundkörper hindurch eine elektrisch leitende Verbindung zwischen einem Innenraum des Gehäuses und einem Außenraum herzustellen. Das Leitungselement ist hermetisch gegen den Grundkörper abgedichtet und weist ein Cermet auf. Ferner verfügt das Leitungselement über eine Verbindungsschicht, die ein Metall beinhaltet. Die Verbindungsschicht schließt an eine drahtähnliche Struktur an, d.h. die Verbindungsschicht befindet sich zwischen dem Cermet aufweisenden Leitungselement und der drahtähnlichen Struktur. Dabei erstreckt sich die Verbindungsschicht ausschließlich über die Fläche des distalen Endes des Leitungselements, das heißt die Verbindungsschicht ist nicht in Kontakt mit dem keramischen Grundkörper.

Bekannte Verbindungsschichten, auch Kontaktpads, bestehen oft aus reinem Metall, bevorzugt aus einem Edelmetall oder einer Edelmetall-Legierung. Eine haftfeste stoffschlüssige Verbindung zwischen einer reinen Metallschicht und einem keramischen Körper ist dabei schwierig darstellbar.

Die WO 2013/019458 A1 offenbart hermetische Durchführungen für implantierbare medizinische Vorrichtungen beinhaltend einen Isolator aus einem keramischen Material, einen Leiter aus einem elektrisch leitfähigen Material und ein Kontaktpad. Das Kontaktpad kann aus mehreren Schichten bestehen. Der Leiter beinhaltet ein Cermet und weist an mindestens einem Ende einen verbreiterten Bereich auf. Das Kontaktpad ist mit dem verbreiterten Bereich am Ende des Leiters durch eine gesinterte Verbindung stoffschlüssig verbunden. Dabei schließt der verbreiterte Bereich des Leiters bündig mit der Oberfläche des keramischen Isolators ab. Üblicherweise wird dies dadurch realisiert, dass eine bindemittelhaltige Cermet-Paste mit Hilfe eines Rakels in die Ausnehmung eines schichtartigen keramischen Grünkörpers eingebracht wird. Anschließend wird das Laminat gebrannt, wobei das Bindemittel der Cermet-Paste verbrennt und eine hermetisch dichte, stoffschlüssige gesinterte Verbindung zwischen dem Cermet und der Keramik erzeugt wird. Der verbreiterte Bereich des Leiters kann dabei nicht beliebig breit oder mit einer beliebig großen Ausdehnung gestaltet werden, da der beim Brennen zu erwartende Schrumpf des Verbundwerkstoffs zu einem Brechen des Laminats führen kann.

In vielen Fällen sind jedoch Kontaktpads oder allgemein Kontaktelemente gefordert, die eine breite und/oder komplexe räumliche Ausdehnung entlang des Grundkörpers einer Durchführung oder allgemein einer Kontaktierungsvorrichtung erfordern. Das kann beispielsweise dann der Fall sein, wenn die drahtähnliche Struktur oder allgemein elektrisch leitfähige Struktur nicht an einem der Enden oder im Bereich der Enden eines Leiters oder allgemein Leitungselements ankoppeln soll, sondern an einem von diesem Ende relativ weit entfernten Bereich. Letzteres erfordert eine gewisse räumliche Ausdehnung des für die Verbindung zwischen dem Leitungselement und der elektrisch leitfähigen Struktur verwendeten Kontaktierungselements.

Die US 2017/0296832 A1 offenbart ein Verfahren zur Herstellung einer Verbindung zwischen einem Leitungselement, auch *via,* einer elektrischen Durchführung und eines Leitungsdrahtes, auch *lead,* mit Hilfe eines Kontaktpads. Die Durchführung weist einen keramischen Isolator, ein Platin aufweisendes *via* und ein Kontaktpad auf. Das Kontaktpad ist mit dem *via* elektrisch verbunden und weist Platin auf. Ferner ist das Kontaktpad mit dem Isolator verbunden. Wie vorgenannt sind haftfeste Verbindungen zwischen einem reinen Metall und einem keramischen Isolator nur schwierig darstellbar, was insbesondere in implantierbaren Vorrichtungen ein nicht unerhebliches Sicherheitsrisiko darstellen kann.

Ein Verfahren zur Herstellung einer Durchführung mit verbesserter Adhäsion bzw. Stabilität und Dichtheit ist ferner aus der EP 2 837 446 A1 bekannt.

### ALLGEMEINE BESCHREIBUNG DER ERFINDUNG

Allgemein ist es eine Aufgabe der vorliegenden Erfindung, die vorgenannten Nachteile des Standes der Technik zu überwinden.

Eine weitere Aufgabe der vorliegenden Erfindung liegt darin eine Kontaktierungsvorrichtung bereitzustellen, die eine haftfeste Verbindung zwischen einem Kontaktelement und dem keramischen Teil der Kontaktierungsvorrichtung ermöglicht.

Eine weitere Aufgabe der vorliegenden Erfindung liegt darin eine Kontaktierungsvorrichtung bereitzustellen, die ein von der räumlichen Positionierung des Leitungselements unabhängiges Ankoppeln der elektrisch leitfähigen Struktur über ein Kontaktelement, das elektrisch mit dem Leitungselement verbunden ist, zu ermöglichen.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie, insbesondere der erfindungsgemäßen elektrischen Kontaktierungsvorrichtung und des erfindungsgemäßen Verfahrens, sind ebenso bevorzugt für gleichnamige oder entsprechende Bestandteile der jeweils anderen erfindungsgemäßen Kategorie. Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

In einem ersten Aspekt der Erfindung wird eine elektrische Kontaktierungsvorrichtung für eine medizinische implantierbare Vorrichtung vorgeschlagen, umfassend einen elektrisch isolierenden Grundkörper mit einer ersten und einer zweiten Oberfläche,
wobei der Grundkörper eine Keramik beinhaltet, ein elektrisch leitendes Leitungselement,
das sich von der ersten Oberfläche des Grundkörpers wenigstens teilweise durch den Grundkörper hindurch erstreckt, wobei das Leitungselement ein Cermet beinhaltet und stoffschlüssig durch eine gesinterte Verbindung mit der Keramik des Grundkörpers verbunden ist, ein Kontaktelement beinhaltend ein Metall, wobei das Kontaktelement mit dem Leitungselement elektrisch leitend verbunden ist und mit einer elektrisch leitfähigen Struktur verbindbar ist.

Erfindungsgemäß ist vorgesehen, dass die Kontaktierungsvorrichtung ein Adhäsionselement umfasst, wobei das Adhäsionselement stoffschlüssig mit dem Kontaktelement verbunden ist und wobei das Adhäsionselement einen Haftvermittler beinhaltet, um zumindest mit der ersten Oberfläche des Grundkörpers eine stoffschlüssige Verbindung auszubilden.

Zusätzlich zu dem Kontaktelement, das die Verbindung zur elektrisch leitenden Struktur ermöglicht, verfügt die erfindungsgemäße Kontaktierungsvorrichtung über ein zusätzliches Adhäsionselement. Das Adhäsionselement stellt einen Haftvermittler bereit, der eine haftfeste stoffschlüssige Verbindung zu zumindest der ersten Oberfläche des keramischen Grundkörpers bereitstellt. Erfindungsgemäß weist das Adhäsionselement eine erste Schicht auf, wobei die erste Schicht ein Metall beinhaltet und sich durch eine zweite Schicht hindurch zum Leitungselement hin erstreckt, wobei die zweite Schicht ein dielektrisches Material beinhaltet und zumindest mit der ersten Schicht und der ersten Oberfläche des Grundkörpers stoffschlüssig verbunden ist. Erfindungsgemäß werden ferner das Adhäsionselement auf dem Grundkörper durch einen Sinterschritt 1 aus einem Adhäsionselementvorläufer unter Ausbildung einer stoffschlüssigen Verbindung zumindest zwischen dem Adhäsionselement und der ersten Oberfläche des Grundkörpers erzeugt sowie das Kontaktelement auf dem Adhäsionselement durch einen Sinterschritt 2 aus einem Kontaktelement-Vorläufer unter Ausbildung einer elektrischen Verbindung zwischen dem Kontaktelement und dem Leitungselement und einer stoffschlüssigen Verbindung zwischen dem Kontaktelement und dem Adhäsionselement erzeugt. Das Adhäsionselement kann mindestens zwei Schichten aufweisen, wobei sich die erste Schicht durch die zweite Schicht hindurch erstreckt und dabei das Leitungselement kontaktiert. Dabei weist die erste Schicht ein Metall auf, um eine haftfeste stoffschlüssige Verbindung zu dem Kontaktelement sowie dem Leitungselement, das ein Cermet und dadurch ein Metall beinhaltet, sicherzustellen. Die zweite Schicht beinhaltet ein dielektrisches Material, wobei dieses Material als Haftvermittler eine stoffschlüssige Verbindung zur ersten Oberfläche des keramischen Grundkörpers ermöglicht. Die erste Schicht beinhaltet vorzugsweise ebenfalls einen Haftvermittler, der eine haftfeste stoffschlüssige Verbindung zu der zweiten Schicht ermöglicht. Die zweite Schicht kann sich ausschließlich entlang der ersten Oberfläche des keramischen Grundkörpers erstrecken oder sich alternativ entlang der ersten Oberfläche des keramischen Grundkörpers und einem Ende des Leitungselements erstrecken. Im ersten Fall steht die zweite Schicht des Adhäsionselements in Kontakt mit dem Kontaktelement, der ersten Schicht des Adhäsionselements und dem Leitungselement. Im zweiten Fall steht die zweite Schicht des Adhäsionselements in Kontakt mit dem Kontaktelement sowie der ersten Schicht des Adhäsionselements, nicht jedoch in Kontakt mit dem Leitungselement.

Insbesondere weist die zweite Schicht ein Loch auf, wobei das Loch eine Ausnehmung definiert, durch die sich die erste Schicht zum Leitungselement hin erstreckt.

Eine bevorzugte Ausführungsform der elektrischen Kontaktierungsvorrichtung ist dadurch gekennzeichnet, dass der Haftvermittler eine Keramik, ein amorphes Glas, ein rekristallisierbares Glas oder eine Kombination aus mindestens zwei hiervon beinhaltet. Dabei ist ein rekristallisierbares Glas gegenüber einem amorphen Glas bevorzugt.

Im Sinne der Erfindung ist eine Keramik, ein amorphes Glas und/oder ein rekristallisierbares Glas ein dielektrisches Material.

Vorzugsweise beinhaltet der Haftvermittler kein Glas, oder der Haftvermittler beinhaltet ausschließlich eine Keramik. In diesem Fall weist die erste Schicht des Adhäsionselements kein Glas auf, oder die erste Schicht des Adhäsionselements beinhaltet als Haftvermittler ausschließlich eine Keramik. Weist das Adhäsionselement zwei (oder mehr) Schichten auf, dann weist die erste und die zweite Schicht kein Glas auf, oder der Haftvermittler der ersten und der zweiten Schicht des Adhäsionselements weist kein Glas auf.

Werden besonders hohe Anforderungen an die Biokompatibilität, insbesondere Biostabilität der Kontaktierungsvorrichtung gestellt, beispielsweise bei Verwendung der Kontaktierungsvorrichtung für eine medizinische implantierbare Vorrichtung, ist es von Vorteil wenn das Adhäsionselement frei von Glas ist.

Eine bevorzugte Ausführungsform der elektrischen Kontaktierungsvorrichtung ist dadurch gekennzeichnet, dass das Adhäsionselement wenigstens 10 Gew.-% des Haftvermittlers, bezogen auf das Gesamtgewicht des Adhäsionselements, beinhaltet. Bevorzugt beinhaltet das Adhäsionselement wenigstens 25 Gew.-% Haftvermittler, oder wenigstens 35 Gew.-% Haftvermittler.

In einer Ausführungsform besteht das Adhäsionselement aus Partikeln des Haftvermittlers und des Metalls, die nach Entfernung eines Bindemittels zu einer haftfesten Schicht gesintert werden. Gemäß einer weiteren Ausführungsform weist das Adhäsionselement daher wenigstens 40 Gew.% Metall auf, bevorzugt wenigstens 50 Gew.-%, weiter bevorzugt wenigstens 75 Gew.-%, oder 85 Gew.-%, oder 90 Gew.-%.

Eine bevorzugte Ausführungsform der elektrischen Kontaktierungsvorrichtung ist dadurch gekennzeichnet, dass die erste Schicht ein Cermet beinhaltet.

Die erste Schicht kann ein Cermet beinhalten, wenn das Adhäsionselement ausschließlich eine erste Schicht umfasst, oder wenn das Adhäsionselement eine erste Schicht und eine zweite Schicht umfasst, wobei die erste Schicht ein Metall beinhaltet und sich durch eine zweite Schicht hindurch zum Leitungselement hin erstreckt.

Eine bevorzugte Ausführungsform der elektrischen Kontaktierungsvorrichtung ist dadurch gekennzeichnet, dass das Cermet Aluminiumoxid und Platin beinhaltet.

Für den keramischen Anteil des Cermets wird vorzugsweise Aluminiumoxid ausgewählt, für den Metall-Anteil des Cermets wird vorzugsweise Platin ausgewählt.

Eine bevorzugte Ausführungsform der elektrischen Kontaktierungsvorrichtung ist dadurch gekennzeichnet, dass das amorphe Glas wenigstens 45 Gew.-% Siliciumoxid (SiO₂) beinhaltet.

Eine bevorzugte Ausführungsform der elektrischen Kontaktierungsvorrichtung ist dadurch gekennzeichnet, dass das rekristallisierbare Glas wenigstens 25 Gew.-% Aluminiumoxid (Al₂O₃) und nicht mehr als 30 Gew.-% Siliciumoxid (SiO₂) beinhaltet.

Der angegebene Gehalt bezieht sich jeweils auf das rekristallisierbare Glas im gesinterten oder gebrannten Zustand. Vorzugsweise beinhaltet das rekristallisierbare Glas wenigstens 40 Gew.-% Aluminiumoxid, besonders bevorzugt wenigstens 45 Gew.-% Aluminiumoxid und bevorzugt nicht mehr als 60 Gew.-% Aluminiumoxid. Vorzugsweise beinhaltet das rekristallisierbare Glas wenigstens 10 Gew.-% Siliciumoxid, besonders bevorzugt wenigstens 20 Gew.-% Siliciumoxid und weiter bevorzugt wenigstens 22 Gew.-% Siliciumoxid.

Eine bevorzugte Ausführungsform der elektrischen Kontaktierungsvorrichtung ist dadurch gekennzeichnet, dass das Adhäsionselement mit dem Grundkörper eine oxidische Mischkristall-Schicht ausbildet.

Eine bevorzugte Ausführungsform der elektrischen Kontaktierungsvorrichtung ist dadurch gekennzeichnet, dass das Kontaktelement ein Edelmetall beinhaltet. Als Edelmetalle bevorzugt sind Pt, Au, Pd und Ag oder eine Legierung aus mindestens zwei Metallen daraus.

Für häufig in der Medizintechnik verwendete Drähte aus der Legierung MP35N®, einer Nickel-Kobalt-Chrom-Molybdän-Legierung, werden Kontaktelemente aus Pd bevorzugt.

Eine bevorzugte Ausführungsform der elektrischen Kontaktierungsvorrichtung ist dadurch gekennzeichnet, dass das Kontaktelement als Leiterbahn ausgebildet ist.

Hierunter wird verstanden, dass das Kontaktelement im Wesentlichen zweidimensional entlang der ersten Oberfläche des Grundkörpers verläuft, wobei die Ausdehnung des Kontaktelements in wenigstens einer Richtung wesentlich größer ist als der Durchmesser des Leitungselements. Die räumliche Ausdehnung des Kontaktelements kann wenigstens fünfmal, oder zehnmal, oder hundertmal größer sein als die räumliche Ausdehnung des Leitungselements, insbesondere des Durchmessers des Leitungselements. Dadurch wird eine elektrische Kontaktierung des Leitungselements durch die elektrisch leitfähige Struktur an einer Stelle ermöglicht, die sich relativ weit entfernt von einem Ende des Leitungselements befindet.

In einer weiteren Ausführungsform können sowohl das Adhäsionselement sowie das Kontaktelement als Leiterbahn ausgestaltet sein. Auch ist denkbar, dass das Adhäsionselement als Leiterbahn ausgestaltet ist und das Kontaktelement ein einer Stelle auf das Adhäsionselement derart aufgebracht ist, dass das Kontaktelement in seiner räumlichen Ausdehnung wesentlich kleiner ist als die räumliche Ausdehnung des Adhäsionselements.

Eine bevorzugte Ausführungsform der elektrischen Kontaktierungsvorrichtung ist dadurch gekennzeichnet, dass das Kontaktelement als Kontaktpad ausgebildet ist. Hierunter wird verstanden, dass die räumliche Ausdehnung des Kontaktelements in wenigstens einer Richtung nicht wesentlich größer ist als die räumliche Ausdehnung des Leitungselements. Die räumliche Ausdehnung des Kontaktelements in einer Richtung kann dabei höchstens 4 mal, oder 2 mal, oder 1,5 mal größer sein als die räumliche Ausdehnung des Leitungselements, insbesondere der Durchmesser des Leitungselements.

Eine bevorzugte Ausführungsform der elektrischen Kontaktierungsvorrichtung ist dadurch gekennzeichnet, dass das Kontaktelement ein durch elektrochemische Abscheidung erzeugtes Metall beinhaltet.

Da zumindest eine der Schichten des Adhäsionselements ein Metall beinhaltet, ist das Adhäsionselement elektrisch leitend. Dadurch wird eine galvanische oder stromlose Abscheidung des Kontaktelements auf dem Adhäsionselement ermöglicht. Der Vorteil dieser Vorgehensweise besteht darin, dass die Abscheidung des Kontaktelements ausschließlich im Bereich des Adhäsionselements stattfindet, da nur diese Fläche, nicht jedoch die erste Oberfläche des Grundkörpers, elektrisch leitend sind. Dadurch entspricht die Geometrie des Kontaktelements exakt der Geometrie des Adhäsionselements. Zusätzlich kann auf die Anwendung eines maskengebenden Verfahrens verzichtet werden.

Eine bevorzugte Ausführungsform der elektrischen Kontaktierungsvorrichtung ist dadurch gekennzeichnet, dass sich das Leitungselement von der ersten Oberfläche des Grundkörpers durch den Grundkörper hindurch zur zweiten Oberfläche des Grundkörpers erstreckt.

Dadurch, dass sich das Leitungselement von der ersten Oberfläche des Grundkörpers durch den Grundkörper hindurch zur zweiten Oberfläche des Grundkörpers erstreckt, kann die Kontaktierungsvorrichtung als Durchführung in einer medizinischen Vorrichtung verwendet werden. Vorzugsweise liegen sich die erste und zweite Oberfläche des Grundkörpers als parallele Oberflächen gegenüber. Dabei kann sich das Leitungselement als gerade Linie, stufenförmig oder Serpentinen-artig von der ersten Oberfläche zur zweiten Oberfläche des Grundkörpers erstrecken. Das Leitungselement weist ein ersten Ende im Bereich der ersten Oberfläche des Grundkörpers und ein zweites Ende im Bereich der zweiten Oberfläche des Grundkörpers auf.

Eine bevorzugte Ausführungsform der elektrischen Kontaktierungsvorrichtung ist dadurch gekennzeichnet, dass die Keramik des Grundkörpers wenigstens 90 Gew.-% Aluminiumoxid (Al₂O₃)aufweist, bevorzugt wenigstens 99 Gew.-% Aluminiumoxid aufweist, besonders bevorzugt wenigstens 99,9 Gew.-% Aluminiumoxid aufweist.

Bevorzugt weisen das Kontaktelement oder das Adhäsionselement oder beide kombiniert eine Schichtdicke von 0,1 µm bis 100 µm auf.

Ist das Kontaktelement als Kontaktpad ausgestaltet, liegt der Durchmesser typischerweise in einem Bereich zwischen 50 µm und 1000 µm. Ist das Kontaktelement als Leiterbahn ausgestaltet, liegt die Breite der Leiterbahn typischerweise in einem Bereich zwischen 50 µm und 1000 µm.

Einen Beitrag zur Erfüllung mindestens einer der erfindungsgemäßen Aufgaben leistet ferner eine medizinische implantierbare Vorrichtung, aufweisend eine Kontaktierungsvorrichtung nach einer der vorhergehenden Ausführungsformen.

Insbesondere kann die elektrische Kontaktierungsvorrichtung oder die medizinisch implantierbare Vorrichtung in einem Herzschrittmacher, einem Defibrillator, einem Neurostimulator, einem Cochlear-Implantat, einem Glucose-Monitor oder in einer implantierbaren Infusionspumpe eingesetzt werden.

Einen Beitrag zur Erfüllung mindestens einer der erfindungsgemäßen Aufgaben leistet ferner ein Verfahren zur Herstellung einer elektrischen Kontaktierungsvorrichtung für eine medizinische implantierbare Vorrichtung, wobei das Verfahren die folgenden Schritte umfasst:
a. Bereitstellen eines elektrisch isolierenden Grundkörpers mit einer ersten und einer zweiten Oberfläche,
   wobei der Grundkörper eine Keramik beinhaltet, und
   wobei der Grundkörper ein elektrisch leitendes Leitungselement beinhaltet,
   das sich von der ersten Oberfläche des Grundkörpers wenigstens teilweise durch den Grundkörper hindurch erstreckt,
   wobei das Leitungselement ein Cermet beinhaltet und stoffschlüssig durch eine gesinterte Verbindung mit der Keramik des Grundkörpers verbunden ist,
b. Erzeugen eines einen Haftvermittler aufweisenden Adhäsionselements auf dem Grundkörpers durch einen Sinterschritt 1 aus einem Adhäsionselement-Vorläufer unter Ausbildung einer stoffschlüssigen Verbindung zumindest zwischen dem Adhäsionselement und der ersten Oberfläche des Grundkörpers,
c. Erzeugen eines ein Metall beinhaltenden Kontaktelements auf dem Adhäsionselement durch einen Sinterschritt 2 aus einem Kontaktelement-Vorläufer unter Ausbildung einer elektrischen Verbindung zwischen dem Kontaktelement und dem Leitungselement und einer stoffschlüssigen Verbindung zwischen dem Kontaktelement und dem Adhäsionselement.

Gemäß dem erfindungsgemäßen Verfahren wird zunächst ein eine Keramik beinhaltender Grundkörper bereitgestellt, der ein von diesem Grundkörper umschlossenes elektrisches Leitungselement umfasst, welches sich von der ersten Seite des Grundkörpers zumindest teilweise durch den Grundkörper hindurch erstreckt. Vorzugsweise erstreckt sich das Leitungselement von der ersten Seite des Grundkörpers vollständig durch den Grundkörper zur zweiten Seite hindurch, wobei sich die beiden Seiten des Grundkörpers parallel gegenüberliegen. Das Leitungselement weist ein Cermet auf und ist stoffschlüssig und hermetisch dicht mit dem Grundkörper verbunden.

Anschließend wird aus einem Adhäsionselement-Vorläufer in einem ersten Sinterschritt (Sinterschritt 1) ein Adhäsionselement erzeugt. Das Adhäsionselement weist einen Haftvermittler auf. Das Adhäsionselement bildet durch den Sinter-Prozess zumindest mit dem Grundkörper im Bereich der ersten Oberfläche eine stoffschlüssige Verbindung aus. In einer bevorzugten Ausführungsform bildet das Adhäsionselement mit dem Grundkörper sowie mit dem Leitungselement im Bereich des ersten Endes des Leitungselements eine stoffschlüssige Verbindung aus.

In einem folgenden Schritt wird aus einem Kontaktelement-Vorläufer in einem zweiten Sinterschritt (Sinterschritt 2) ein Kontaktelement erzeugt. Das Kontaktelement weist ein Metall auf. Durch den Sinter-Prozess bildet das Kontaktelement mit dem Adhäsionselement eine stoffschlüssige Verbindung aus. Das das Adhäsionselement zumindest in einer Schicht, die in Kontakt mit dem Leitungselement ist, ein Metall beinhaltet, wird durch den Sinterschritt 2 eine elektrische Verbindung zwischen dem Kontaktelement und dem Leitungselement ausgebildet.

Eine bevorzugte Ausführungsform des erfindungsgemäßen Verfahrens ist dadurch gekennzeichnet, dass der Adhäsionselement-Vorläufer zumindest eine Paste 1 beinhaltet, wobei die Paste 1 zumindest ein Metall, einen Haftvermittler und ein Bindemittel beinhaltet.

Die Paste 1 wird vorzugsweise zum Aufbringen der ersten Schicht des Adhäsionselements verwendet. Nach dem Aufbringen der Paste wird diese gesintert oder gebrannt, wodurch die erste Schicht des Adhäsionselements erzeugt wird. Die Paste 1 ist vorzugsweise eine elektrisch leitfähige Paste, so dass auch die gesinterte erste Schicht elektrisch leitfähig ist.

Eine bevorzugte Ausführungsform des erfindungsgemäßen Verfahrens ist somit dadurch gekennzeichnet, dass im Sinterschritt 1 die Paste 1 auf den Grundkörper aufgebracht und gesintert wird, wobei das Adhäsionselement unter Ausbildung einer stoffschlüssigen Verbindung mit dem Leitungselement und der ersten Oberfläche des Grundkörpers gebildet wird.

Dabei wird die Paste 1 vorzugsweise in Kontakt mit dem keramischen Grundkörper sowie der Oberfläche des Leitungselements gebracht.

Eine weitere bevorzugte Ausführungsform des erfindungsgemäßen Verfahrens ist dadurch gekennzeichnet, dass der Adhäsionselement-Vorläufer eine Paste 2 beinhaltet, wobei die Paste 2 zumindest einen Haftvermittler und ein Bindemittel beinhaltet.

Die Paste 2 wird vorzugsweise zum Aufbringen der zweiten Schicht des Adhäsionselements verwendet. Nach dem Aufbringen der Paste wird diese gesintert oder gebrannt, wodurch die zweite Schicht des Adhäsionselements erzeugt wird. Vorzugsweise handelt es sich bei der Paste 2 um eine elektrisch nicht leitfähige oder dielektrische Paste, die keine elektrisch leitfähigen Metallpartikel beinhaltet.

Eine bevorzugte Ausführungsform des erfindungsgemäßen Verfahrens ist dadurch gekennzeichnet, dass der Kontaktelement-Vorläufer eine Paste 3 beinhaltet, wobei die Paste 3 zumindest ein Metall und ein Bindemittel beinhaltet.

Zum Erzeugen des Kontaktelements wird vorzugsweise eine Paste 3 verwendet, die zumindest ein Metall und ein Bindemittel beinhaltet. Nach dem Aufbringen der Paste auf das bereits gesinterte Adhäsionselement wird die Paste 3 gesintert oder gebrannt und dadurch das Kontaktelement erzeugt. Es ist daneben auch denkbar dass sowohl die Paste 1 und/oder Paste 2 und die Paste 3 nacheinander aufgebracht werden und anschließend zusammen gesintert werden, wodurch das Adhäsionselement und das Kontaktelement in einem einzigen Sinterschritt erzeugt werden. Die Paste 3 weist bevorzugt außer dem Metall nur Bestandteile auf, die durch Erhitzen auf die jeweilige Sintertemperatur entfernt werden, also unter den Sinterbedingungen flüchtig sind, so dass das Kontaktelement nach dem Sintern ausschließlich oder im Wesentlichen ein Metall aufweist. Unter den Sinterbedingungen flüchtige Bestandteile sind insbesondere ein Bindemittel, ein Lösungsmittel, ein Tensid oder ein Additiv.

Eine bevorzugte Ausführungsform des erfindungsgemäßen Verfahrens ist dadurch gekennzeichnet, dass im Sinterschritt 1 zunächst die Paste 2 auf den Grundkörper derart aufgebracht und gesintert wird dass zumindest ein Teil einer freiliegenden Oberfläche des Leitungselements nicht von der Paste 2 bedeckt wird, und anschließend die Paste 1 auf die gesinterte Paste 2 aufgebracht und gesintert wird, wobei das Adhäsionselement unter Ausbildung einer stoffschlüssigen Verbindung mit dem Leitungselement und der ersten Oberfläche des Grundkörpers gebildet wird.

In diesem Fall wird zunächst eine dielektrische Paste derart auf den keramischen Grundkörper aufgebracht, dass eine definierte Fläche des Leitungselements freiliegend bleibt, so dass das Leitungselement später über die Paste 1, die elektrisch leitfähige Metallpartikel aufweist, elektrisch kontaktierbar ist. Vorzugsweise wird zunächst die dielektrische Paste 2 aufgebracht und gesintert. Anschließend wird die elektrisch leitfähige Paste 1 auf die gesinterte zweite Schicht so aufgebracht dass Paste 1 den von der zweiten Schicht freigelassenen Bereich füllt und in Kontakt mit der freiliegenden Oberfläche des Leitungselements kommt. Anschließend wird Paste 1 gesintert, so dass auch die erste Schicht des Adhäsionselements erzeugt wird. Damit wurde im Sinterschritt 1 ein zweischichtiges Adhäsionselement erzeugt.

### Keramik

Eine Keramik im Sinne der Erfindung kann jede Keramik sein, die der Fachmann für den erfindungsgemäßen Einsatz auswählen würde. Die Keramik ist bevorzugt ausgewählt aus der Gruppe bestehend aus einer Oxidkeramik, einer Silikatkeramik, einer Nichtoxid-Keramik oder einer Mischung aus mindestens zwei davon.

Die Oxidkeramik ist bevorzugt ausgewählt aus der Gruppe bestehend aus einem Metalloxid, einem Halbmetalloxid oder einer Mischung davon. Das Metall des Metalloxids kann ausgewählt sein aus der Gruppe bestehend aus Aluminium, Beryllium, Barium, Calcium, Magnesium, Natrium, Kalium, Eisen, Zirkonium, Titan oder einer Mischung von mindestens zwei davon. Das Metalloxid ist bevorzugt ausgewählt aus der Gruppe bestehend aus Aluminiumoxid (Al₂O₃), Magnesiumoxid (MgO), Zirkoniumoxid (ZrO₂), Yttriumoxid (Y₂O₃), Aluminiumtitanat (Al₂TiO₅), einer Piezokeramik wie Blei-Zirkonat (PbZrO₃), Blei-Titanat (PbTiO₃) sowie Blei-Zirkonat-Titanat (PZT) oder einer Mischung von mindestens zwei hiervon. Das Halbmetall des Halbmetalloxids ist bevorzugt ausgewählt aus der Gruppe bestehend aus Bor, Silicium, Arsen, Tellur oder einer Mischung von mindestens zwei davon. Eine weitere bevorzugte Oxidkeramik beinhaltet eines ausgewählt aus der Gruppe bestehend aus Zirkoniumoxid verstärktes Aluminiumoxid (ZTA - Zirconia Toughened Aluminum - Al₂O₃/ZrO₂), Yttrium verstärktes Zirkoniumoxid (Y-TZP), Barium(Zr, Ti)oxid, Barium(Ce, Ti)oxid oder eine Kombination aus mindestens zwei davon.

Die Silikatkeramik ist bevorzugt ausgewählt aus der Gruppe bestehend aus einem Steatit (Mg₃[Si₄O₁₀(OH)₂]), Cordierit (Mg, Fe²⁺)₂(Al₂Si)[Al₂Si₄O₁₈]), Mullit (Al₂Al₂₊₂ₓSi₂₋₂ₓO₁₀₋ₓ mit x = Sauerstoffleerstellen pro Elementarzelle), Feldspat (Ba, Ca, Na, K, NH₄)(Al, B, Si)₄O₈) oder einer Mischung aus mindestens zwei davon.

Die Nichtoxid-Keramik kann ausgewählt sein aus der Gruppe bestehend aus einem Carbid, einem Nitrid oder einer Mischung daraus. Das Carbid kann ausgewählt sein aus der Gruppe bestehend aus Siliciumcarbid (SiC), Borcarbid (B₄C), Titancarbid (TiC), Wolframcarbid, Ze-mentit (Fe₃C). Das Nitrid kann ausgewählt sein aus der Gruppe bestehend aus Siliciumnitrid (Si₃N₄), Aluminiumnitrid (AIN), Titannitrid (TiN), Siliciumaluminiumoxinitrid (SIALON) oder einer Mischung aus mindestens zwei davon. Eine weitere bevorzugte Nichtoxid-Keramik ist Natrium-Kalium-Niobat.

### Amorphes Glas

Erfindungsgemäß wird unter einem amorphen Glas ein Gemisch aus wenigstens zwei unterschiedlichen Metall- und/oder Halbmetalloxiden verstanden, das in festem Zustand bei Raumtemperatur eine amorphe Struktur aufweist und nach dem Erhitzen auf eine bestimmte Sintertemperatur und anschließendem Abkühlen im amorphen Zustand verbleibt. Nach dem Erhitzen über die Glasübergangstemperatur hinaus und anschließendem Abkühlen bildet ein amorphes Glas somit keine Kristalle oder kristallinen Bereiche. Die Viskosität eines amorphen Glases sinkt mit zunehmender Temperatur, das heißt das Material beginnt zu fließen wenn die angewandte Temperatur beim Sinterprozess signifikant über der Glasübergangstemperatur liegt. Vor dem Sintern oder Brennen kann das Gemisch Zusatzstoffe enthalten. Zusatzstoffe können ein Bindemittel, ein Lösungsmittel, ein Tensid, ein Additiv oder ein anderer Hilfsstoff oder eine Kombination aus wenigstens zwei daraus sein. Die Komponenten des amorphen Glases sind bevorzugt ausgewählt aus der Gruppe bestehend aus einem Metalloxid, einem Halbmetalloxid oder einer Mischung davon. Vorzugsweise weist das amorphe Glas wenigstens 3 unterschiedliche Metall- und/oder Halbmetalloxide auf, bevorzugt wenigstens 5 unterschiedliche Metall- und/oder Halbmetalloxide, weiter bevorzugt wenigstens 10 unterschiedliche Metall- und/oder Halbmetalloxide. Vorzugsweise ist das Halbmetalloxid Siliciumoxid (SiO₂). Vorzugsweise sind die Metalloxide ausgewählt aus der Gruppe bestehend aus Bariumoxid (BaO), Aluminiumoxid (Al₂O₃), Cadmiumoxid (CdO), Natriumoxid (Na₂O), Calciumoxid (CaO), Strontiumoxid (SrO), Zinkoxid (ZnO), Magnesiumoxid (MgO), Eisenoxid (Fe₂O₃), Kupferoxid (CuO), Kaliumoxid (K₂O) und Bleioxid (PbO). Bevorzugt beinhaltet das amorphe Glas wenigstens 45 Gew.-% Siliciumoxid, bevorzugt wenigstens 50 Gew.-% Siliciumoxid, jeweils bezogen auf die Zusammensetzung im gesinterten oder gebrannten Zustand. Bevorzugt beinhaltet das amorphe Glas wenigstens 15 Gew.-% Bariumoxid, besonders bevorzugt wenigstens 25 Gew.-% Bariumoxid und weiter bevorzugt wenigstens 30 Gew.-% Bariumoxid, jeweils bezogen auf die Zusammensetzung im gesinterten oder gebrannten Zustand. Bevorzugt beinhaltet das amorphe Glas wenigstens 5 Gew.-% Aluminiumoxid, besonders bevorzugt wenigstens 10 Gew.-% Aluminiumoxid und weiter bevorzugt wenigstens 15 Gew.-% Aluminiumoxid, jeweils bezogen auf die Zusammensetzung im gesinterten oder gebrannten Zustand.

### Rekristallisierbares Glas

Erfindungsgemäß wird unter einem rekristallisierbaren Glas ein Gemisch aus wenigstens zwei unterschiedlichen Metall- und/oder Halbmetalloxiden verstanden, das in festem Zustand bei Raumtemperatur eine amorphe Struktur aufweist, jedoch nach dem Erhitzen auf eine bestimmte Sintertemperatur und anschließendem Abkühlen wenigstens teilweise Kristalle oder kristalline Strukturen ausbildet. Dadurch ist das rekristiallisierbare Glas gegenüber hohen Temperaturen stabil und weist bei höheren Temperaturen deutlich viskoser, das heißt zähflüssiger, als vergleichbare nicht rekristallisierbare Materialien. Vor dem Sintern oder Brennen kann das Gemisch Zusatzstoffe enthalten. Zusatzstoffe können ein Bindemittel, ein Lösungsmittel, ein Tensid, ein Additiv oder ein anderer Hilfsstoff oder eine Kombination von wenigstens zwei daraus sein. Die Komponenten des rekristallisierbaren Glases sind bevorzugt ausgewählt aus der Gruppe bestehend aus einem Metalloxid, einem Halbmetalloxid oder einer Mischung davon. Vorzugsweise weist das rekristallisierbare Glas wenigstens 3 unterschiedliche Metall- und/oder Halbmetalloxide auf, bevorzugt wenigstens 5 unterschiedliche Metall- und/oder Halbmetalloxide, weiter bevorzugt wenigstens 10 unterschiedliche Metall- und/oder Halbmetalloxide. Vorzugsweise sind die Halbmetalloxide ausgewählt aus der Gruppe bestehend aus Siliciumoxid (SiO₂) und Boroxid (B₂O₃). Vorzugsweise sind die Metalloxide ausgewählt aus der Gruppe bestehend aus Aluminiumoxid (Al₂O₃), Calciumoxid (CaO), Titanoxid (TiO₂), Zinkoxid (ZnO), Magnesiumoxid (MgO), Cadmiumoxid (CdO), Natriumoxid (Na₂O), Eisenoxid (Fe₂O₃), Zirkoniumoxid (ZrO₂) und Chromoxid (Cr₂O₃). Bevorzugt beinhaltet das rekristallisierbare Glas wenigstens 25 Gew.-% Aluminiumoxid, bevorzugt wenigstens 40 Gew.-% Aluminiumoxid, weiter bevorzugt wenigstens 45 Gew.-% Aluminiumoxid, und bevorzugt nicht mehr als 60 Gew.-% Aluminiumoxid, jeweils bezogen auf die Zusammensetzung im gesinterten oder gebrannten Zustand. Bevorzugt beinhaltet das rekristallisierbare Glas wenigstens 10 Gew.-% Siliciumoxid, bevorzugt wenigstens 20 Gew.-% Siliciumoxid, weiter bevorzugt wenigstens 22 Gew.-% Siliciumoxid, und bevorzugt nicht mehr als 30 Gew.-% Siliciumoxid, jeweils bezogen auf die Zusammensetzung im gesinterten oder gebrannten Zustand. Bevorzugt beinhaltet das rekristallisierbare Glas wenigstens 10 Gew.-% Calciumoxid, bevorzugt wenigstens 18 Gew.-% Calciumoxid, weiter bevorzugt wenigstens 20 Gew.-% Calciumoxid, und bevorzugt nicht mehr als 40 Gew.-% Calciumoxid, jeweils bezogen auf die Zusammensetzung im gesinterten oder gebrannten Zustand. Ferner beinhaltet das rekristallisierbare Glas bevorzugt wenigstens 1 Gew.-% und nicht mehr 4 Gew.-% eines Metall- oder Halbmetalloxids ausgewählt aus der Gruppe Boroxid, Titanoxid, Zinkoxid, Magnesiumoxid und Cadmiumoxid oder eine Mischung aus wenigstens zwei davon.

### Cermet

Erfindungsgemäß wird als "Cermet" ein Verbundwerkstoff aus einem oder mehreren keramischen Werkstoffen in mindestens einer metallischen Matrix oder ein Verbundwerkstoff aus einem oder mehreren metallischen Werkstoffen in mindestens einer keramischen Matrix oder beides bezeichnet. Zur Herstellung eines Cermets kann beispielsweise ein Gemisch aus mindestens einem keramischen Pulver und mindestens einem metallischen Pulver verwendet werden, welches beispielsweise mindestens mit einem Bindemittel versetzt werden kann. Das bzw. die keramischen Pulver des Cermets weisen vorzugsweise eine mittlere Korngröße von weniger als 10 µm, bevorzugt weniger als 5 µm, besonders bevorzugt weniger als 3 µm auf. Das bzw. die metallischen Pulver des Cermets weisen vorzugsweise eine mittlere Korngröße von weniger als 15 µm, bevorzugt weniger als 10 µm, besonders bevorzugt weniger als 5 µm auf. Als mittlere Korngröße wird dabei insbesondere der Medianwert oder D₅₀-Wert der Korngrößenverteilung angesehen. Der D₅₀-Wert beschreibt jenen Wert, bei dem 50 % der Körner des keramischen Pulvers und/oder des metallischen Pulvers feiner sind als der D₅₀-Wert. Ein bevorzugtes Cermet weist eine hohe spezifische Leitfähigkeit auf, die bevorzugt mindestens 1 S/m, weiter bevorzugt mindestens 100 S/m, weiter bevorzugt mindestens 103 S/m, weiter bevorzugter mindestens 104 S/m , noch weiter bevorzugt mindestens 105 S/m, und am weitesten bevorzugt mindestens 106 S/m beträgt.

Die mindestens eine keramische Komponente eines erfindungsgemäßen Cermets beinhaltet bevorzugt eine Keramik. Die mindestens eine metallische Komponente eines erfindungsgemäßen Cermets beinhaltet bevorzugt eines ausgewählt aus der Gruppe beste-hend aus Platin, Iridium, Niob, Palladium, Eisen, Edelstahl, einer Kobalt-Chrom-Legierung, Molybdän, Tantal, Wolfram, Titan, Kobalt und Zirkonium oder Kombination von mindestens zwei davon. Dabei ist eine bevorzugte Kombination eine Legierung. Ein bevorzugter Edelstahl ist ein Edelstahl 316L. Eine elektrisch leitfähige Verbindung stellt sich im Cermet in der Regel dann ein, wenn der Metallgehalt über der sogenannten Perkolationsschwelle liegt, bei der die Metallpartikel im gesinterten Cermet mindestens punktuell miteinander verbunden sind, so dass eine elektrische Leitung ermöglicht wird. Dazu sollte der Metallgehalt erfahrungsgemäß, abhängig von der Materialauswahl, mindestens 25 Vol.-% betragen, bevorzugt mindestens 32 Vol.-%, weiter bevorzugt mindestens 38 Vol.-%, jeweils bezogen auf das gesamte Volumen des Cermets.

### Stoffschlüssige, gesinterte Verbindung

Der Grundkörper, das Leitungselement, das Adhäsionselement und das Kontaktelement sind durch eine stoffschlüssige, gesinterte Verbindung miteinander verbunden. Stoffschlüssig hat die Bedeutung, dass die beiden zu verbindenden Teile nach dem Verbinden eine Einheit bilden und die Verbindung selbst eine Festigkeit aufweist, die mindestens der einer der beiden Teile entsprechen kann. Dies kann zur Folge haben, dass die verbundenen Teile bei mechanischer Belastung oder Druckbelastung nicht an der Verbindungsstelle auseinander brechen, sondern an einer anderen Stelle der beiden verbundenen Teile. Hierdurch kann gewährleistet werden, dass die Verbindung genauso oder weniger porös oder gas- oder feuchtigkeitsdurchlässig ist wie die zu verbindenden Teile selbst. In diesem Fall wird auch von einer hermetisch dichten Verbindung gesprochen.

Unter einem Sintern, einem Sinterprozess oder co-Sintern wird im Rahmen der vorliegenden Erfindung allgemein ein Verfahren zur Herstellung von Werkstoffen oder Werkstücken verstanden, bei welchem pulverförmige, insbesondere eines ausgewählt aus der Gruppe bestehend aus feinkörnige Stoffe, keramische Stoffe und metallische Stoffe oder eine Kombination aus mindestens zwei davon erhitzt und dadurch verbunden werden. Dieser Prozess kann ohne äußeren Druck auf den zu erhitzenden Stoff erfolgen oder kann insbesondere unter erhöhtem Druck auf den zu erhitzenden Stoff erfolgen, beispielsweise unter einem Druck von mindestens 2 bar, vorzugsweise höheren Drücken, beispielsweise Drücken von mindestens 10 bar, insbesondere mindestens 100 bar oder sogar mindestens 1000 bar. Der Prozess kann insbesondere vollständig oder teilweise bei Temperaturen unterhalb der Schmelztemperatur der pulverförmigen Werkstoffe erfolgen, beispielsweise bei Temperaturen von 700°C bis 1400 °C. Der Prozess kann insbesondere vollständig oder teilweise in einem Werkzeug oder einer Form oder beides durchgeführt werden, so dass mit dem Sinterprozesses eine Formgebung verbunden werden kann. Neben den pulverförmigen Werkstoffen kann ein Ausgangsmaterial für den Sinterprozess weitere Werkstoffe umfassen, beispielsweise ein oder mehrere Bindemittel, aber auch ein oder mehrere Lösungsmittel, Tenside, Additive oder andere Hilfsstoffe oder eine Kombination aus wenigstens zwei davon. Der Sinterprozess kann in einem Schritt oder auch in mehreren Schritten erfolgen, wobei dem Sinterprozess beispielsweise weitere Schritte vorgelagert sein können, beispielsweise ein oder mehrere Formgebungsschritte oder ein oder mehrere Entbinderungsschritte oder beides. Das Sintern bzw. der Sinterprozess entspricht somit einem Brennprozess. Der Sinterprozess, insbesondere für ein Cermet, kann vergleichbar zu einem üblicherweise für homogene Pulver verwendeten Sinterprozess ablaufen. Beispielsweise kann unter hoher Temperatur und ggf. hohem Druck das Material beim Sintervorgang verdichtet werden, so dass das Cermet nahezu dicht ist, oder eine höchstens geschlossene Porosität aufweist. Cermets zeichnen sich in der Regel durch eine besonders hohe Härte und Verschleißfestigkeit aus.

### Bindemittel

Bevorzugte Bindemittel sind solche, die zum Erhalten einer Zusammensetzung mit geeigneter Stabilität, Eignung zum Drucken, Viskosität und Sinterverhalten beitragen. Bindemittel sind dem Fachmann bekannt. Alle Bindemittel, welche dem Fachmann als geeignet für einen erfindungsgemäßen Einsatz erscheinen, können als solche eingesetzt werden. Bevorzugte Bindemittel sind Harze. Weitere bevorzugte Bindemittel sind polymerische Bindemittel, monomerische Bindemittel und Bindemittel aus einer Kombination aus Polymeren und Monomeren. Polymerische Bindemittel können auch Co-Polymere sein, wobei mindestens zwei monomerische Einheiten in einem einzelnen Molekül beinhaltet sind. Bevorzugte polymerische Bindemittel beinhalten eine funktionelle Gruppe in der Hauptkette des Polymers, außerhalb der Hauptkette, oder in der Hauptkette und außerhalb der Hauptkette. Bevorzugte Bindemittel mit einer funktionellen Gruppe in der Hauptkette sind Polyester, substituierte Polyester, Polycarbonate, substituierte Polycarbonate, Polymere mit einer cyclischen Gruppe in der Hauptkette, Polyzucker, substituierte Polyzucker, Polyurethane, substituierte Polyurethane, Polyamide, substituierte Polyamide, Phenolharze, substituierte Phenolharze, Co-Polymere der Monomere von einem oder mehreren der vorgenannten Polymeren, optional mit anderen Co- Monomeren, oder eine Kombination aus mindestens zwei davon. Bevorzugte Polymere mit einer cyclischen Gruppe in der Hauptkette sind Polyvinylbutylate (PVB) und deren Derivate, und Poly-Terpineol und dessen Derivate, oder Mischungen daraus. Bevorzugte Polyzucker sind Zellulose und Alkylderivate davon, bevorzugt Methylzellulose, Ethylzellulose, Propylzellulose, Butylzellulose, und deren Derivate, und Mischungen aus mindestens zwei davon. Bevorzugte Polymere mit einer funktionellen Gruppe außerhalb ihrer Hauptkette sind solche mit einer Amidgruppe, mit einer Säuregruppe und/oder einer Estergruppe (auch Acrylharze genannt), oder Polymere mit einer Kombination der vorgenannten funktionellen Gruppen. Bevorzugte Polymere mit einer Amidgruppe außerhalb der Hauptkette sind Polyvinyl-Pyrolidon (PVP) und dessen Derivate. Bevorzugte Polymer mit einer Säuregruppe und/oder einer Estergruppe außerhalb der Hauptkette sind Polyacrylsäure und ihre Derivate, Polymethacrylat (PMA) und dessen Derivate, und Polymethylmethacrylat (PMMA) und dessen Derivate, oder Kombinationen aus mindestens zwei davon. Bevorzugte monomerische Bindemittel sind ethylenglycolbasierte Monomere, Terpineol-Harze, und Harzderivate, oder eine Kombination aus mindestens zwei davon. Bevorzugte ethylenglycolbasierte monomerische Bindemittel haben eine Ethergruppe, ein Estergruppe, oder beides. Bevorzugte Ethergruppen sind dabei Methyl-, Ethyl-, Propyl-, Butyl-, Pentyl-, Hexyl-, und höhere Alkylethergruppen. Bevorzugte Estergruppen sind Acetat und dessen Alkylderivate, bevorzugt Ethylenglycol-Monobutylether-Monoacetat, oder eine Mischung aus den Vorgenannten. Besonders bevorzugte Bindemittel sind Alkylzellulose, bevorzugt Ethylzellulose, und deren Derivate, und Mischungen daraus mit anderen Bindemitteln ausgewählt aus den oben aufgeführten oder anderen.

### Biokompatibles Material

Bevorzugt handelt es sich um erfindungsgemäß verwendete Materialien um biokompatible Materialien. Biokompatible Materialien sind ausgewählt aus der Gruppe bestehend aus biotolerant, bioinert und bioaktiv oder einer Kombination aus mindestens zwei davon.

### AUSFÜHRUNGSBEISPIELE

Die Erfindung wird im Folgenden durch Zeichnungen und Ausführungsbeispiele genauer dargestellt, wobei die Zeichnungen und Ausführungsbeispiele keine Beschränkung der Erfindung bedeuten. Die Zeichnungen sind, sofern nicht anders angegeben, nicht maßstabsgetreu.

Es zeigen
- Figur 1): eine Querschnittdarstellung einer erfindungsgemäßen Kontaktierungsvorrichtung;
- Figur 2a): eine Querschnittsdarstellung einer weiteren Ausführungsform der erfindungsgemäßen Kontaktierungsvorrichtung;
- Figur 2b): eine Querschnittsdarstellung einer weiteren Ausführungsform der erfindungsgemäßen Kontaktierungsvorrichtung;
- Figur 3): ein Ablaufdiagramm des erfindungsgemäßen Verfahrens.

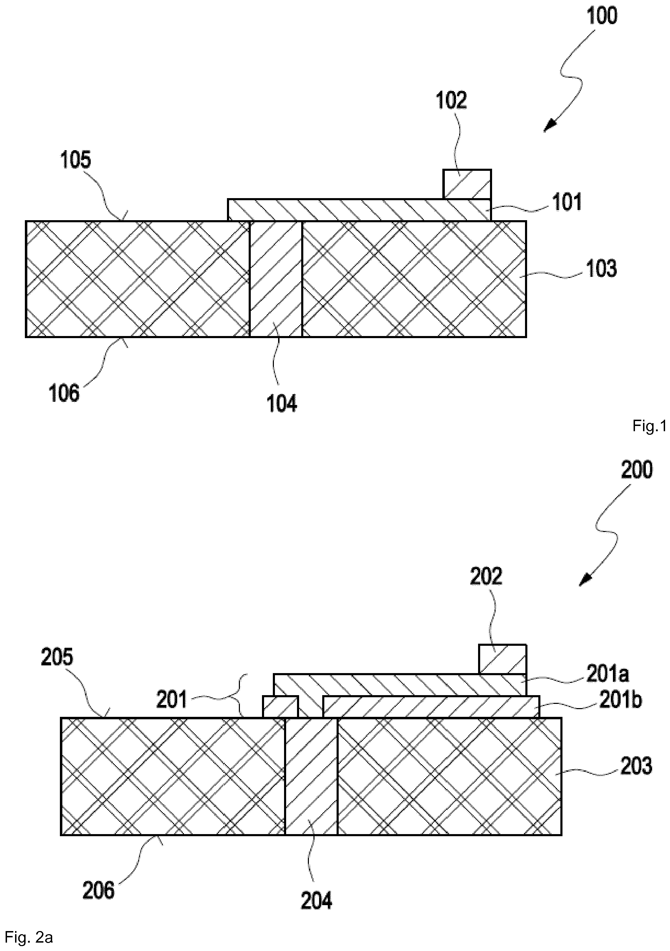

Figur 1 zeigt einen Querschnitt einer elektrischen Kontaktierungsvorrichtung (100) gemäß einer ersten Ausführungsform der Erfindung. Die Kontaktierungsvorrichtung eignet sich beispielsweise als elektrische Durchführung von einem inneren Teil eines Gehäuses einer medizinisch implantierbaren Vorrichtung zu einem äußeren Teil, das heißt der Körperflüssigkeits-Seite. Die Kontaktierungsvorrichtung (100) weist einen elektrisch isolierenden Grundkörper (103) mit einer ersten Oberfläche (105) und einer zweiten Oberfläche (106) auf. Der Grundkörper besteht aus hochreinem Aluminiumoxid. Von der ersten Oberfläche (105) zur zweiten Oberfläche (106) des Grundkörpers (103) erstreckt sich ein elektrisch leitendes Leitungselement (104). Das Leitungselement (104) beinhaltet ein Cermet aus gesinterten Platin- und Aluminiumoxid-Partikeln, wobei der Platin-Anteil ca. 40 Vol.-% und der Aluminiumoxid-Anteil ca. 60 Vol.-% bezogen auf den Cermet im gesinterten Zustand beträgt. Der Cermet des Leitungselements (104) ist durch eine stoffschlüssige, gesinterte Verbindung mit dem Grundkörper (103) verbunden. Die Kontaktierungsvorrichtung (100) beinhaltet weiterhin ein Adhäsionselement (101), das durch eine stoffschlüssige, gesinterte Verbindung mit dem Grundkörper (103), dem Leitungselement (104) sowie einem Kontaktelement (102) verbunden ist. Das Adhäsionselement (101) weist ein Cermet aus Platin- und Aluminiumoxid-Partikeln auf. Die Platin-Partikel dienen vornehmlich der elektrischen Verbindung zwischen dem Leitungselement (104) und dem Kontaktelement (102), dienen aber auch der Adhäsion zu dem Leitungselement (104) sowie zu dem Kontaktelement (102) durch Diffusion der Metallpartikel der einzelnen Elemente im Zuge des Sinterprozesses. Das Kontaktelement (102) besteht aus Platin. Die Aluminiumoxid-Partikel wirken als Haftvermittler in Bezug auf den Grundkörper (103) und sorgen damit für eine haftfeste Verbindung zwischen dem Adhäsionselement (101) und dem Grundkörper (103). Das Kontaktelement (102) ist durch eine stoffschlüssige, gesinterte Verbindung mit dem Adhäsionselement (101) verbunden, was auch für die Verbindung zwischen dem Adhäsionselement (102) und der ersten Oberfläche (105) des Grundkörpers (103) und der Oberfläche des Leitungselements (104) gilt. Im gezeigten Beispiel erstreckt sich das Adhäsionselement (101) über einen Großteil der ersten Oberfläche (105) des Grundkörpers (103), so dass das Kontaktelement (102) an einer Position versetzt zu einem Ende des Leitungselements (104) platziert werden kann. Dadurch wird erreicht, dass die Kontaktierung mit Hilfe einer elektrisch leitfähigen Struktur (nicht gezeigt) am Kontaktelement (102) variabel gestaltet werden kann, unabhängig von der Position des Leitungselements (104) im Grundkörper (103) der Kontaktierungsvorrichtung (100).

Figur 2a zeigt einen Querschnitt einer elektrischen Kontaktierungsvorrichtung (200) gemäß einer weiteren Ausführungsform der Erfindung. Die Kontaktierungsvorrichtung eignet sich beispielsweise als elektrische Durchführung von einem inneren Teil eines Gehäuses einer medizinisch implantierbaren Vorrichtung zu einem äußeren Teil, das heißt der Körperflüssigkeits-Seite. Die Kontaktierungsvorrichtung (200) weist einen elektrisch isolierenden Grundkörper (203) mit einer ersten Oberfläche (205) und einer zweiten Oberfläche (206) auf. Der Grundkörper besteht aus hochreinem Aluminiumoxid. Von der ersten Oberfläche (205) zur zweiten Oberfläche (206) des Grundkörpers (203) erstreckt sich ein elektrisch leitendes Leitungselement (204). Das Leitungselement (204) beinhaltet ein Cermet aus gesinterten Platin- und Aluminiumoxid-Partikeln, wobei der Platin-Anteil ca. 40 Vol.-% und der Aluminiumoxid-Anteil ca. 60 Vol.-% bezogen auf den Cermet im gesinterten Zustand beträgt. Der Cermet des Leitungselements (204) ist durch eine stoffschlüssige, gesinterte Verbindung mit dem Grundkörper (203) verbunden. Die Kontaktierungsvorrichtung (200) beinhaltet weiterhin ein Adhäsionselement (201) bestehend aus einer ersten Schicht (201a) und einer zweiten Schicht (201b). Die erste Schicht (201a) des Adhäsionselements erstreckt sich durch die zweite Schicht (201b) hindurch hin zum Leitungselement (204). Die erste Schicht (201a) beinhaltet ein Cermet aus gesinterten Platin- und Aluminiumoxid-Partikeln. Die Platinpartikel dienen vornehmlich der Herstellung einer elektrischen Verbindung zwischen dem Leitungselement (204) und einem Kontaktelement (202). Weiterhin verbessern die Platinpartikel des Cermets der ersten Schicht (201a) des Adhäsionselements (201) die Adhäsion zum Kontaktelement (202) und zum Leitungselement (204) durch Diffusion der Metallpartikel der einzelnen Elemente im Zuge des Sinterprozesses. Das Kontaktelement (202) besteht aus Platin. Die zweite Schicht (201b) des Adhäsionselements (201) weist als Haftvermittler ein dielektrisches Material auf. Das dielektrische Material sorgt für eine haftfeste Verbindung zum Grundkörper (203) aus Aluminiumoxid und zur ersten Schicht (201a), da die erste Schicht anteilig Aluminiumoxid aufweist. Im gezeigten Beispiel kommt als dielektrisches Material ein rekristallisierbares Glas aus 48 Gew.-% Aluminiumoxid (Al₂O₃), 23 Gew.-% Siliciumoxid (SiO₂), 21 Gew.-% Calciumoxid (CaO), 4 Gew.-% Boroxid (B₂O₃), jeweils 1 Gew.-% Zinkoxid (ZnO), Magnesiumoxid (MgO) und Cadmiumoxid (CdO) sowie Spuren von Natrium-, Eisen, Zirkonium- und Chromoxiden, bezogen auf den gesinterten Zustand des rekristallisierbaren Glases, zum Einsatz. Die zweite Schicht (201b) weist dabei ein Loch als Ausnehmung auf, durch die sich die erste Schicht (201a) zum Leitungselement (204) hin erstreckt. Beispielsweise ist die Ausnehmung, das heißt das Loch der zweiten Schicht, als kreisrundes Loch ausgebildet. Bei allen in Figur 2a gezeigten Verbindungen zwischen den Schichten des Adhäsionselements (201a, 201b), des Kontaktelements (202), des Leitungselements (204) und der ersten Oberfläche (205) des Grundkörpers (203) handelt es sich um stoffschlüssige, gesinterte Verbindungen. Dabei besteht zwischen der zweiten Schicht (201b) des Adhäsionselements (202) eine Verbindung mit sowohl der ersten Oberfläche (205) des Grundkörpers (203) sowie der Oberfläche des Leitungselements (204). Im gezeigten Beispiel erstreckt sich das Adhäsionselement (201) über einen Großteil der ersten Oberfläche (205) des Grundkörpers (203), so dass das Kontaktelement (202) an einer Position versetzt zu einem Ende des Leitungselements (204) platziert werden kann. Dadurch wird erreicht dass die Kontaktierung mit Hilfe einer elektrisch leitfähigen Struktur (nicht gezeigt) am Kontaktelement (202) variabel gestaltet werden kann, unabhängig von der Position des Leitungselements (204) im Grundkörper (203) der Kontaktierungsvorrichtung (200).

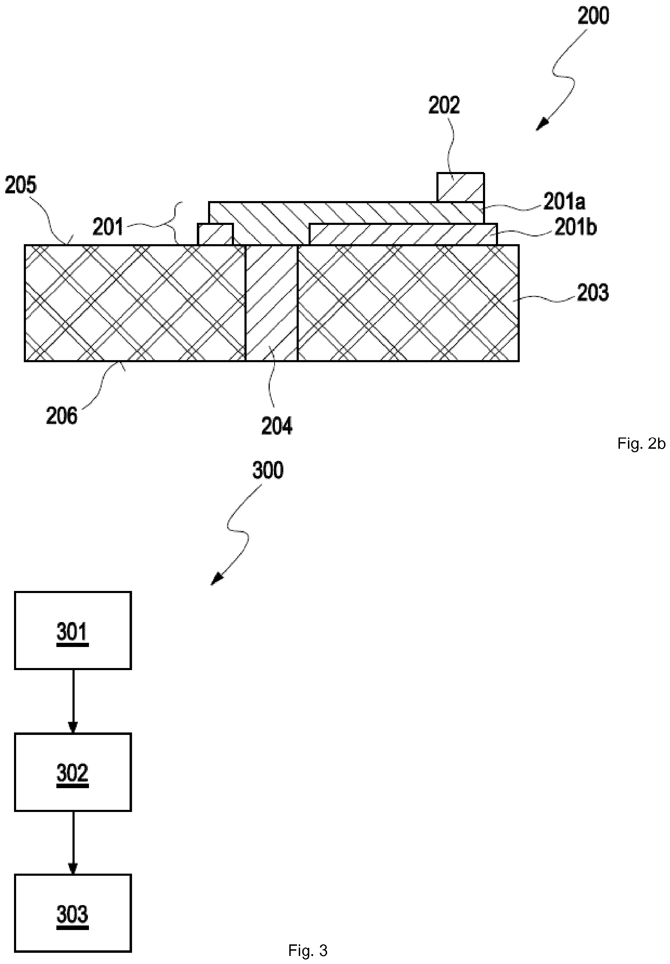

Figur 2b zeigt einen Querschnitt einer elektrischen Kontaktierungsvorrichtung (200) gemäß einer weiteren Ausführungsform der Erfindung. Dabei unterscheidet sich die Kontaktierungsvorrichtung (200) von Figur 2b von der Kontaktierungsvorrichtung (200) von Figur 2a dadurch, dass die zweite Schicht des Adhäsionselements (201b) nicht mit der Oberfläche des Leitungselements (204) verbunden ist. Die zweite Schicht des Adhäsionselements ist lediglich mit der ersten Oberfläche (205) des Grundkörpers (203) sowie der ersten Schicht (201a) des Adhäsionselements (201) verbunden. Die zweite Schicht (201b) weist ein Loch als Ausnehmung auf, durch die sich die zweite Schicht (201a) des Adhäsionselements zum Kontaktelement als auch zur ersten Oberfläche (205) des Grundkörpers (203) hin erstreckt, das heißt die zweite Schicht bildet eine stoffschlüssige Verbindung zum Leitungselement (204) als auch zum Grundkörper (205) aus. Beispielsweise ist die Ausnehmung, das heißt das Loch der zweiten Schicht, als kreisrundes Loch ausgebildet.

Figur 3 zeigt einen schematischen Ablauf des erfindungsgemäßen Verfahrens (300) zur Herstellung einer erfindungsgemäßen Kontaktierungsvorrichtung. Dabei wird in einem ersten Schritt (301) zunächst ein elektrisch isolierender Grundkörper mit einer ersten und einer zweiten Oberfläche bereitgestellt, wobei der Grundkörper eine Keramik und ein in den Grundkörper integriertes elektrisch leitendes Leitungselement beinhaltet, das sich von der ersten Oberfläche des Grundkörpers durch den Grundkörper hindurch erstreckt, wobei das Leitungselement ein Cermet beinhaltet und stoffschlüssig durch eine gesinterte Verbindung mit der Keramik des Grundkörpers verbunden ist. Der Grundkörper mit dem Leitungselement ist durch einen Mullit-Schichtaufbau gekennzeichnet, d.h. der Aufbau des Grundkörpers mit Leitungselement erfolgt durch Laminieren von mit Öffnungen versehenen Aluminiumoxid-Schichten und Füllen der Öffnungen mit einer Cermet-Paste, die anschließend mit dem Aluminiumoxid co-gesintert wird. Das Aufbringen des Adhäsions- und Kontaktelements erfolgt nach dem Co-Sintern des Laminats. Entsprechend wird in einem zweiten Schritt (302) ein Adhäsionselement-Vorläufer aufgebracht. Der Adhäsionselement-Vorläufer beinhaltet eine oder zwei Pasten, je nach Schichtaufbau. Durch Sintern des Adhäsionselement-Vorläufers in einem Sinterschritt 1 wird eine stoffschlüssig Verbindung zwischen dem Adhäsionselement und dem Grundkörper und optional der Oberfläche des Leitungselements erzeugt. Dabei kann der Sinterschritt 1 zwei voneinander getrennte Sinterschritte umfassen, in dem Fall dass das Adhäsionselement mehr als eine Schicht umfasst. In diesem Fall wird zunächst die zweite Schicht des Adhäsionselement-Vorläufers aufgetragen und gesintert, bevor die erste Schicht des Adhäsionselement-Vorläufers aufgetragen und gesintert wird. In einem weiteren Schritt (303) erfolgt das Erzeugen des Kontaktelements durch Auftragen eines Kontaktelements-Vorläufers und Sintern in einem Sinterschritt 2, wobei eine elektrische Verbindung zwischen dem Kontaktelement und dem Leitungselement sowie eine stoffschlüssige Verbindung zwischen dem Kontaktelement und dem Adhäsionselement erzeugt wird.

In den folgenden Ausführungsbeispielen wird die Herstellung einer elektrischen Kontaktierungsvorrichtung näher beschrieben.

### Vorbereitung keramischer Grünkörperfolien

Keramische Grünkörperfolien wurden als Keramikvorläufer für den isolierenden Grundkörper eingesetzt. Hierzu wurden 99 Gew.-% reine Al₂O₃ - Folien (**Maryland Ceramic & Steatite Company Inc.**) der Dicke 400 µm verwendet. Proben der Grünkörperfolien wurden zu 100 mm x 100 mm Quadraten zugschnitten. Etwa kreisrunde Löcher mit einem Durchmesser von 400 µm wurden mit einem mechanischen Stanzwerkzeug (für 400 µm Durchmesser in einer automatisierten Stanze in die Folienproben gestanzt. Derart wurden mindestens 4 Folienproben vorbereitet.

### Füllen

Die wie oben vorbereiteten Löcher wurden mit Hilfe einer Druck-Schablone und einem EKRA Microtronic II-Drucker (Modell M2H) mit Cermetpaste gefüllt.

Für die Cermetpaste wurde Platinpulver und Al₂O₃-Pulver mit einem organischen Bindemittel gemischt und mit einer 3-Walzenmühle homogenisiert. So erhaltene Pasten hatten Viskositäten in einem Bereich von 250 bis 500 Pa*s (gemessen mit einem Haake Rheostress 6000 Rheometer bei 25 °C) und eine Mahlfeinheit (fineness of grind - FoG) von weniger als 20 µm. Die Rheologie der Pasten war geeignet für den anschließenden Schablonendruck.

Die Dicke der Schablone betrug 100 µm. Die Öffnungen der Schablone hatten die gleichen Abmessungen und Positionen wie die Löcher, welche wie oben beschrieben in die Grünkörperfolie gestanzt worden waren. Die Druckparameter waren 50 N Rakeldruck, Rakelgeschwindigkeit vorwärts 25 mm/s, Rakelgeschwindigkeit rückwärts 25 mm/s und Absprung (snap off) 0,0 mm. Der Rakelkreis wurde so eingestellt, dass Pastenmaterial sowohl bei der Vorwärtsbewegung als auch bei der Rückwärtsbewegung eingebracht wurde.

10 Minuten nach dem Füllen der Proben wurden diese in einen Trockner HHG-2 (von BTU International Inc.) eingebracht und dort für 10 Minuten bei 75 °C getrocknet.

Zum vollständigen Füllen des Lochs der Folie wurden weitere Füllschritte mit der Cermetpaste durchgeführt. Es wurden 1 bis 5 Folienproben durch mehrfaches Ausführen des obigen Füllschrittes mit der Cermetpaste vollständig gefüllt.

### Laminieren der Grünkörperfolien

Es werden 4 Lagen Grünkörperfolie mit wie oben beschrieben gefüllten Löchern mit einem Metallausrichtungswerkzeug gestapelt und in einem isostatisch verpresst.

### Sintern

Das wie oben erhaltene Laminat von Grünkörperfolien wurde in einem Hochtemperatur-Kammerofen mit einer Kammergröße von 200 mm x 250 mm x 200 mm gebrannt, um die einzelnen Schichten und Cermet-Füllungen zu sintern. Der Sintervorgang erfolgte unter atmosphärischen Normalbedingungen. Die Temperatur wurde langsam von 25 auf 450 °C bei erhöht. Dann wurde die Temperatur bei 450 °C für 1 h konstant gehalten, um die organischen Komponenten in dem Grünkörper-Laminat auszutreiben. Anschließend wurde die Temperatur rasch auf eine Maximaltemperatur in einem Bereich von 1510 bis 1560 °C erhöht und bei diesem Wert für eine Haltedauer in einem Bereich von 1 bis 2 Stunden konstant gehalten. Danach wurde die Temperatur bei einer Kühlrate von 500 °C/h oder der natürlichen Kühlrate, welche langsamer war, auf Raumtemperatur abgesenkt.

Es wurden gesinterte Formkörper mit einem Volumenanteil von 40 Vol.-% bis 45 Vol.-% Platin im Cermet erhalten.

### Nachbehandlung

Nach dem Brennen wurden die Proben abgeschliffen und mit einem Laser auf die gewünschten Abmessungen zugeschnitten.

Die gesinterten Proben wurden auf beiden Seiten auf eine Dicke von 1,0 bis 1,1 mm geschliffen. Aus den geschliffenen Proben wurden einzelne Bereiche mit Hilfe eines Laserschneidverfahrens herausgetrennt. Es wurden Bereiche erhalten, die je Probe 5 Doppelreihen von Cermet-Leitungselementen enthielten.

### Erzeugen des Adhäsions- und Kontaktelements auf den Proben

### Beispiel 1

Eine wie oben beschrieben hergestellte Probe wurde als Substrat verwendet. Eine Cermet-Paste beinhaltend Platinpulver und Al₂O₃-Pulver und einem organischen Bindemittel wurde mit Hilfe eines Siebdruckverfahrens (Maschenweite 200 Mesh/ 30 µm Schichtdicke der Emulsion) auf die Oberfläche des Grundkörpers und des Leitungselements aufgedruckt. Die Probe wurde bei 1450 °C (Haltezeit: 2 h) in einem Hochtemperatur-Kammerofen unter normaler Atmosphäre gesintert. Eine zusätzliche Schicht einer Glas- und Keramik-freien Platinpaste, nur bestehend aus Platinpartikeln und einem organischen Vehikel, wurde mit Hilfe eines Siebdruckverfahrens (Maschenweite 200 Mesh/ 30 µm Schichtdicke der Emulsion) auf das gesinterte Adhäsionselement aufgedruckt. Die Probe wurde bei 1150 °C oder 1300 °C bei einer Haltezeit von 40 min ein weiteres Mal gesintert.

### Beispiel 2

Eine wie oben beschrieben hergestellte Probe wurde als Substrat verwendet. Eine dielektrische, ein rekristallisierendes Glas und ein Bindemittel aufweisende Paste wurde mit Hilfe eines Siebdruckverfahrens (Maschenweite 200 Mesh/ 30 µm Schichtdicke der Emulsion) auf die Oberfläche des Grundkörpers und des Leitungselements aufgedruckt, wobei eine Teilfläche des Leitungselements nicht bedruckt, das heißt frei gelassen, wurde. Die Probe wurde bei 850 °C für 1 h (Haltezeit bei 850 °C: 10 min) in einem Hochtemperatur-Kammerofen unter normaler Atmosphäre gesintert. Eine Cermet-Paste beinhaltend ein Platinpulver und ein Al₂O₃-Pulver und ein organisches Bindemittel auf Ethylcellulose-Basis wurde mit Hilfe eines Siebdruckverfahrens (Maschenweite 200 Mesh/ 30 µm Schichtdicke der Emulsion) auf die Oberfläche der zuerst aufgebrachten Schicht aus rekristallisierbarem Glas aufgedruckt. Die Probe wurde bei 1550 °C (Haltezeit: 0,5 h) in einem Hochtemperatur-Kammerofen unter normaler Atmosphäre gesintert. Eine zusätzliche Schicht einer Glas- und Keramik-freien Platinpaste, nur bestehend aus Platinpartikeln und einem organischen Vehikel, wurde mit Hilfe eines Siebdruckverfahrens (Maschenweite 200 Mesh/ 30 µm Schichtdicke der Emulsion) auf das gesinterte Adhäsionselement aufgedruckt, wobei auch der vorher freigelassene Bereich des Leitungselements bedruckt wurde. Die Probe wurde bei 1150 °C oder 1300 °C ein weiteres Mal gesintert (Haltezeit bei 1150 °C oder 1300 °C: 40 min).

### TESTMETHODEN

### Adhäsion

Die Adhäsion der gesinterten Schichten (Adhäsions- und Kontaktelement) wurde mit Hilfe des sogenannten *tape pull* Test geprüft. Dafür wurden die gemäß dem oben erläuterten Verfahren bedruckten Bereiche mit einem Stück Tesa®-Film beklebt. Um sicher zu stellen, dass der Film vollständig am bedruckten Bereich haftet, wurde dieser mit Hilfe eines Stücks Gummis fest angedrückt. Anschließend wurde der Film in einer langsamen und kontinuierlichen Bewegung (Abzieh-Geschwindigkeit ca. 1 Sekunde pro zoll) wieder entfernt. Die Probe und der Film wurden anschließend optisch begutachtet. Keine der hergestellten Proben ließ erkennen, dass ein Teil des bedruckten Bereichs am Film haften blieb.

Als Alternative zu diesem Test wurden die Adhäsion der gesinterten Schichten in den Proben im sogenannten *scratch test* geprüft. Dafür wurde für einige Sekunden energisch mit der Spitze einer Pinzette aus Edelstahl über die bedruckten Bereiche gekratzt und die Probe anschließend optisch inspiziert. Keine der hergestellten Proben ließ erkennen, dass ein Teil des bedruckten Bereichs durch die Bearbeitung mit der Pinzette entfernt werden konnte.

### Lötbarkeit

Die Lötbarkeit der gedruckten Schichten, insbesondere bezüglich des Kontaktelements, beschreibt die Fähigkeit der jeweils gedruckten Schicht mit einem Lötmetall benetzt zu werden. Nur in dem Fall dass eine Benetzung beobachtet wird ist das Anlöten eines Drahtes an das Kontaktelement mit Hilfe einer Lötverbindung unter Standardbedingungen möglich. Zum Testen der Lötbarkeit wurde eine Ecke der Probe für 5 Sekunden in ein Gefäß mit bei 255 °C verflüssigtem Lötmetall (Typ SAC305 der Firma AIM) eingetaucht, so dass eine Fläche der Keramik des Grundkörpers, eine Fläche des Adhäsionselements (Platin-Cermet oder rekristallisierbares Glas) und eine Fläche des Kontaktelements (Platin) mit dem Lötmetall in Kontakt kamen. Dabei wurde beobachtet, dass wie erwünscht ausschließlich das Kontaktelement aus Platin mit dem Lötmetall benetzt wurde. Der Platin-Cermet und das Aluminiumoxid des Grundkörpers zeigten keine Benetzbarkeit durch das Lötmetall.

### LISTE DER BEZUGSZEICHEN

- **100, 200**: Kontaktierungsvorrichtung
- **101, 201**: Adhäsionselement
- **102, 202**: Kontaktelement
- **103, 203**: Grundkörper
- **104, 204**: Leitungselement
- **105, 205**: Erste Oberfläche des Grundkörpers
- **106, 206**: Zweite Oberfläche des Grundkörpers
- **201a**: Erste Schicht des Adhäsionselements
- **201b**: Zweite Schicht des Adhäsionselements
- **300**: Verfahren zur Herstellung der Kontaktierungsvorrichtung
- **301**: Verfahrensschritt 1
- **302**: Verfahrensschritt 2
- **303**: Verfahrensschritt 3

## Patentansprüche

1. Elektrische Kontaktierungsvorrichtung (100, 200) für eine medizinische implantierbare Vorrichtung, umfassend
einen elektrisch isolierenden Grundkörper (103, 203) mit einer ersten (105, 205) und einer zweiten Oberfläche (106, 206),
wobei der Grundkörper eine Keramik beinhaltet,
ein elektrisch leitendes Leitungselement (104, 204),
das sich von der ersten Oberfläche des Grundkörpers wenigstens teilweise durch den Grundkörper hindurch erstreckt,
wobei das Leitungselement ein Cermet beinhaltet und stoffschlüssig durch eine gesinterte Verbindung mit der Keramik des Grundkörpers verbunden ist,
ein Kontaktelement (102, 202) beinhaltend ein Metall, wobei das Kontaktelement mit dem Leitungselement elektrisch leitend verbunden ist und mit einer elektrisch leitfähigen Struktur verbindbar ist,
**dadurch gekennzeichnet, dass**
die Kontaktierungsvorrichtung ein Adhäsionselement (101, 201) umfasst, wobei
das Adhäsionselement auf dem Grundkörper durch einen Sinterschritt 1 aus einem Adhäsionselementvorläufer unter Ausbildung einer stoffschlüssigen Verbindung zumindest zwischen dem Adhäsionselement und der ersten Oberfläche des Grundkörpers erzeugt wurde und wobei
das Adhäsionselement einen Haftvermittler beinhaltet, um zumindest mit der ersten Oberfläche des Grundkörpers eine stoffschlüssige Verbindung auszubilden, und das Adhäsionselement eine erste Schicht (201a) aufweist, wobei die erste Schicht ein Metall beinhaltet und sich durch eine zweite Schicht hindurch zum Leitungselement hin erstreckt, wobei die zweite Schicht (201b) ein dielektrisches Material beinhaltet und zumindest mit der ersten Schicht und der ersten Oberfläche des Grundkörpers stoffschlüssig verbunden ist
und wobei das Kontaktelement auf dem Adhäsionselement durch einen Sinterschritt 2 aus einem Kontaktelement-Vorläufer unter Ausbildung einer elektrischen Verbindung zwischen dem Kontaktelement und dem Leitungselement und einer stoffschlüssigen Verbindung zwischen dem Kontaktelement und dem Adhäsionselement erzeugt wurde.

2. Elektrische Kontaktierungsvorrichtung nach einem der vorangehenden Ansprüche, **dadurch gekennzeichnet, dass** der Haftvermittler eine Keramik, ein amorphes Glas, ein rekristallisierbares Glas oder eine Kombination aus mindestens zwei hiervon beinhaltet.

3. Elektrische Kontaktierungsvorrichtung nach einem der vorangehenden Ansprüche, **dadurch gekennzeichnet, dass** die erste Schicht ein Cermet beinhaltet.

4. Elektrische Kontaktierungsvorrichtung nach Anspruch 2 oder 3, **dadurch gekennzeichnet, dass** das amorphe Glas wenigstens 45 Gew.-% Siliciumoxid (SiO₂) beinhaltet.

5. Elektrische Kontaktierungsvorrichtung nach einem der Ansprüche 2 bis 4,
**dadurch gekennzeichnet, dass** das rekristallisierbare Glas wenigstens 25 Gew.% Aluminiumoxid (Al₂O₃) und nicht mehr als 30 Gew.-% Siliciumoxid (SiO₂) beinhaltet.

6. Elektrische Kontaktierungsvorrichtung nach einem der vorangehenden Ansprüche, **dadurch gekennzeichnet, dass** das Adhäsionselements mit dem Grundkörper eine oxidische Mischkristall-Schicht ausbildet.

7. Elektrische Kontaktierungsvorrichtung nach einem der vorhergehenden Ansprüche, **dadurch gekennzeichnet, dass** sich das Leitungselement von der ersten Oberfläche des Grundkörpers durch den Grundkörper hindurch zur zweiten Oberfläche des Grundkörpers erstreckt.

8. Medizinische implantierbare Vorrichtung, aufweisend eine Kontaktierungsvorrichtung nach einem der vorhergehenden Ansprüche 1 bis 7.

9. Herzschrittmacher, Defibrillator, Neurostimulator, Cochlea-Implantat, Glucose-Monitor oder implantierbare Infusionspumpe aufweisend eine elektrische Kontaktierungsvorrichtung nach einem der Ansprüche 1 bis 7 oder eine medizinische implantierbare Vorrichtung nach Anspruch 8.

10. Verfahren zur Herstellung einer elektrischen Kontaktierungsvorrichtung für eine medizinische implantierbare Vorrichtung, wobei das Verfahren die folgenden Schritte umfasst:
a. Bereitstellen eines elektrisch isolierenden Grundkörpers mit einer ersten und einer zweiten Oberfläche,
wobei der Grundkörper eine Keramik beinhaltet, und
wobei der Grundkörper ein elektrisch leitendes Leitungselement beinhaltet, das sich von der ersten Oberfläche des Grundkörpers wenigstens teilweise durch den Grundkörper hindurch erstreckt,
wobei das Leitungselement ein Cermet beinhaltet und stoffschlüssig durch eine gesinterte Verbindung mit der Keramik des Grundkörpers verbunden ist (301),
b. Erzeugen eines einen Haftvermittler aufweisenden Adhäsionselements auf dem Grundkörpers durch einen Sinterschritt 1 aus einem Adhäsionselement-Vorläufer unter Ausbildung einer stoffschlüssigen Verbindung zumindest zwischen dem Adhäsionselement und der ersten Oberfläche des Grundkörpers (302),
c. Erzeugen eines ein Metall beinhaltenden Kontaktelements auf dem Adhäsionselement durch einen Sinterschritt 2 aus einem Kontaktelement-Vorläufer unter Ausbildung einer elektrischen Verbindung zwischen dem Kontaktelement und dem Leitungselement und einer stoffschlüssigen Verbindung zwischen dem Kontaktelement und dem Adhäsionselement (303).

11. Verfahren nach Anspruch 10, **dadurch gekennzeichnet, dass** der Adhäsionselement-Vorläufer zumindest eine Paste 1 beinhaltet, wobei die Paste 1 zumindest ein Metall, einen Haftvermittler und ein Bindemittel beinhaltet.

12. Verfahren nach Anspruch 10 oder 11, **dadurch gekennzeichnet, dass** der Adhäsionselement-Vorläufer eine Paste 2 beinhaltet, wobei die Paste 2 zumindest einen Haftvermittler und ein Bindemittel beinhaltet.

13. Verfahren nach Anspruch 11, **dadurch gekennzeichnet, dass** im Sinterschritt 1 die Paste 1 auf den Grundkörper aufgebracht und gesintert wird, wobei das Adhäsionselement unter Ausbildung einer stoffschlüssigen Verbindung mit dem Leitungselement und der ersten Oberfläche des Grundkörpers gebildet wird.

14. Verfahren nach Anspruch 12, **dadurch gekennzeichnet, dass** im Sinterschritt 1 zunächst die Paste 2 auf den Grundkörper derart aufgebracht und gesintert wird, dass zumindest ein Teil einer freiliegenden Oberfläche des Leitungselements nicht von der Paste 2 bedeckt wird, und anschließend die Paste 1 auf die gesinterte Paste 2 aufgebracht und gesintert wird, wobei das Adhäsionselement unter Ausbildung einer stoffschlüssigen Verbindung mit dem Leitungselement und der ersten Oberfläche des Grundkörpers gebildet wird.

15. Elektrische Kontaktierungsvorrichtung für eine medizinische implantierbare Vorrichtung, erhältlich durch ein Verfahren nach einem der Ansprüche 10 bis 14.

16. Verwendung einer elektrischen Kontaktierungsvorrichtung nach einem der Ansprüche 1 bis 7 für eine medizinische implantierbare Vorrichtung nach Anspruch 8 oder für einen Herzschrittmacher, einen Defibrillator, einen Neurostimulator, ein Cochlea-Implantat, einen Glucose-Monitor oder eine implantierbare Infusionspumpe nach Anspruch 9.

## Claims

1. An electrical contacting device (100, 200) for a medical implantable device, comprising
an electrically insulating base body (103, 203) having a first (105, 205) and a second surface (106, 206),
wherein the base body includes a ceramics,
an electroconductive conducting element (104, 204),
which extends from the first surface of the base body at least partially through the base body,
wherein the conducting element includes a cermet and is connected to the ceramics of the base body by means of a substance-to-substance bond by a sintered connection,
a contact element (102, 202) including a metal, wherein
the contact element is connected to the conducting element in an electroconductive manner and can be connected to an electroconductive structure,
**characterised in that**
the contacting device comprises an adhesion element (101, 201), wherein the adhesion element was generated on the base body by a sintering step 1 from an adhesion element precursor by forming a substance-to-substance bond at least between the adhesion element and the first surface of the base body and wherein
the adhesion element includes an adhesion promoter in order to form a substance-to-substance bond at least with the first surface of the base body, and the adhesion element has a first layer (201a), wherein the first layer includes a metal and extends through a second layer to the conducting element, wherein the second layer (201b) includes a dielectric material and is connected by means of a substance-to-substance bond to at least the first layer and the first surface of the base body,
and wherein the contact element was generated on the adhesion element by a sintering step 2 from a contact element precursor by forming an electrical connection between the contact element and the conducting element and a substance-to-substance bond between the contact element and the adhesion element.

2. The electrical contacting device according to any one of the preceding claims, **characterised in that** the adhesion promoter includes a ceramics, an amorphous glass, a recrystallisable glass, or a combination or at least two of them.

3. The electrical contacting device according to any one of the preceding claims, **characterised in that** the first layer includes a cermet.

4. The electrical contacting device according to claim 2 or 3, **characterised in that** the amorphous glass includes at least 45% by weight of silicon oxide (SiO₂).

5. The electrical contacting device according to any one of claims 2 to 4, **characterised in that** the recrystallisable glass includes at least 25% by weight of aluminium oxide (Al₂O₃)and not more than 30% by weight of silicon oxide (SiO₂).

6. The electrical contacting device according to any one of the preceding claims, **characterised in that** the adhesion element forms an oxidic mixed crystal layer with the base body.

7. The electrical contacting device according to any one of the preceding claims, **characterised in that** the conducting element extends from the first surface of the base body through the base body to the second surface of the base body.

8. A medical implantable device having a contacting device according to any one of the preceding claims 1 to 7.

9. A cardiac pacemaker, defibrillator, neurostimulator, cochlear implant, glucose monitor or implantable infusion pump having an electrical contacting device according to any one of claims 1 to 7 or a medical implantable device according to claim 8.

10. A method for producing an electrical contacting device for a medical implantable device, wherein the method comprises the following steps:
a. providing an electrically insulating base body having a first and a second surface,
wherein the base body includes a ceramics, and
wherein the base body includes an electroconductive conducting element, which extends from the first surface of the base body at least partially through the base body,
wherein the conducting element includes a cermet and is connected to the ceramics of the base body by means of a substance-to-substance bond by a sintered connection (301),
b. generating an adhesion element having an adhesion promoter on the base body by a sintering step 1 from an adhesion element precursor by forming a substance-to-substance bond at least between the adhesion element and the first surface of the base body (302),
c. generating a contact element including a metal on the adhesion element by a sintering step 2 from a contact element precursor by forming an electrical connection between the contact element and the conducting element and a substance-to-substance bond between the contact element and the adhesion element (303).

11. The method according to claim 10, **characterised in that** the adhesion element precursor includes at least a paste 1, wherein the paste 1 includes at least a metal, an adhesion promoter, and a binding agent.

12. The method according to claim 10 or 11, **characterised in that** the adhesion element precursor includes a paste 2, wherein the paste 2 includes at least an adhesion promoter and a binding agent.

13. The method according to claim 11, **characterised in that** in sintering step 1, the paste 1 is applied to the base body and is sintered, wherein the adhesion element is formed by forming a substance-to-substance bond with the conducting element and the first surface of the base body.

14. The method according to claim 12, **characterised in that** in sintering step 1, the paste 2 is initially applied to the base body and is sintered in such a way that at least a part of an exposed surface of the conducting element is not covered by the paste 2, and the paste 1 is subsequently applied to the sintered paste 2 and is sintered, wherein the adhesion element is formed by forming a substance-to-substance bond with the conducting element and the first surface of the base body.

15. An electrical contacting device for a medical implantable device, obtainable by a method according to any one of claims 10 to 14.

16. Use of an electrical contacting device according to any one of claims 1 to 7 for a medical implantable device aording to claim 8 or for a cardiac pacemaker, a defibrilator, a neurostimulator, a cochlear implant, a glucose monitor, or an implantable infusion pump according to claim 9.

## Revendications

1. Dispositif de connexion électrique (100, 200) pour un dispositif médical implantable, comprenant
un corps de base (103, 203) électriquement isolant avec une première (105, 205) et une deuxième surface (106, 206),
le corps de base contenant une céramique,
un élément de conduction (104, 204) électriquement conducteur qui s'étend depuis la première surface du corps de base au moins en partie à travers le corps de base,
l'élément de conduction contenant un cermet et étant relié matériellement par une liaison frittée avec la céramique du corps de base,
un élément de contact (102, 202) contenant un métal,
l'élément de contact étant relié par conduction électrique avec l'élément de conduction et pouvant être relié avec une structure électriquement conductrice,
**caractérisé en ce que**
le dispositif de connexion comprend un élément d'adhésion (101, 201), l'élément d'adhésion ayant été généré sur le corps de base par une étape de frittage 1 à partir d'un élément d'adhésion antérieur avec la formation d'une liaison matérielle au moins entre l'élément d'adhésion et la première surface du corps de base et
l'élément d'adhésion contenant un agent d'adhérence afin de former une liaison matérielle au moins avec la première surface du corps de base et l'élément d'adhésion présentant une première couche (201a), la première couche contenant un métal et s'étendant vers l'élément de conduction à travers une deuxième couche, la deuxième couche (201b) contenant un matériau diélectrique et étant relié matériellement au moins avec la première couche et la première surface du corps de base
et l'élément de contact ayant été généré sur l'élément d'adhésion par une étape de frittage 2 à partir d'un élément de contact antérieur avec la formation d'une liaison électrique entre l'élément de contact et l'élément de conduction ainsi que d'une liaison matérielle entre l'élément de contact et l'élément d'adhésion.

2. Dispositif de connexion électrique conformément à l'une des revendications précédentes, **caractérisé en ce que** l'agent d'adhérence est une céramique, un verre amorphe, un verre recristallisable ou une combinaison d'au moins deux de ces éléments.

3. Dispositif de connexion électrique conformément à l'une des revendications précédentes, **caractérisé en ce que** la première couche contient un cermet.

4. Dispositif de connexion électrique conformément à la revendication n°2 ou n°3, **caractérisé en ce que** le verre amorphe contient au moins 45 % en poids d'oxyde de silicium (SiO₂).

5. Dispositif de connexion électrique conformément à l'une des revendications n°2 à n°4, **caractérisé en ce que** le verre recristallisable contient au moins 25 % en poids d'oxyde d'aluminium (Al₂O₃)et au plus 30 % en poids d'oxyde de silicium (SiO₂).

6. Dispositif de connexion électrique conformément à l'une des revendications précédentes, **caractérisé en ce que** l'élément d'adhésion forme une couche de cristal mixte oxydique avec le corps de base.

7. Dispositif de connexion électrique conformément à l'une des revendications précédentes, **caractérisé en ce que** l'élément de conduction s'étend depuis la première surface du corps de base vers la deuxième surface du corps de base à travers le corps de base.

8. Dispositif médical implantable, présentant un dispositif de connexion conformément à l'une des revendications précédentes n°1 à n°7.

9. Stimulateur cardiaque, défibrillateur, neurostimulateur, implant cochléaire, moniteur de glucose ou pompe de perfusion implantable présentant un dispositif de connexion électrique conformément à l'une des revendications n°1 à n°7 ou un dispositif médical implantable conformément à la revendication n°8.

10. Procédé pour la fabrication d'un dispositif de connexion électrique un dispositif médical implantable, le procédé comprenant les étapes suivantes :
a. Mise à disposition d'un corps de base électriquement isolant avec une première et une deuxième surface,
le corps de base contenant une céramique et
le corps de base contenant un élément de conduction électriquement conducteur qui s'étend depuis la première surface du corps de base au moins en partie à travers le corps de base,
l'élément de conduction contenant un cermet et étant relié matériellement par une liaison frittée avec la céramique du corps de base (301),
b. Génération d'un élément d'adhésion présentant un agent d'adhérence sur le corps de base par une étape de frittage 1 à partir d'un élément d'adhésion antérieur avec la formation d'une liaison matérielle au moins entre l'élément d'adhésion et la première surface du corps de base (302),
c. Génération d'un élément de contact contenant un métal sur l'élément d'adhésion par une étape de frittage 2 à partir d'un élément de contact antérieur avec la formation d'une liaison électrique entre l'élément de contact et l'élément de conduction ainsi que d'une liaison matérielle entre l'élément de contact et l'élément d'adhésion (303).

11. Procédé conformément à la revendication n°10, **caractérisé en ce que** l'élément d'adhésion antérieur contient au moins une pâte 1, la pâte 1 contenant au moins un métal, un agent d'adhérence et un liant.

12. Procédé conformément à la revendication n°10 ou n°11, **caractérisé en ce que** l'élément d'adhésion antérieur contient une pâte 2, la pâte 2 contenant au moins un agent d'adhérence et un liant.

13. Procédé conformément à la revendication n°11, **caractérisé en ce que** la pâte 1 est appliquée sur le corps de base et frittée dans l'étape de frittage 1, l'élément d'adhésion étant formé avec la formation d'une liaison matérielle avec l'élément de conduction et la première surface du corps de base.

14. Procédé conformément à la revendication n°12, **caractérisé en ce que** la pâte 2 est d'abord appliquée sur le corps de base et frittée dans l'étape de frittage 1 de telle manière à ce qu'au moins une partie d'une surface libre de l'élément de conduction ne soit pas recouverte de la pâte 2, la pâte 1 est ensuite appliquée sur la pâte 2 frittée et frittée, l'élément d'adhésion étant formé avec la formation d'une liaison matérielle avec l'élément de conduction et la première surface du corps de base.

15. Dispositif de connexion électrique pour un dispositif médical implantable, disponible par un procédé conformément à l'une des revendications n°10 à n°14.

16. Utilisation d'un dispositif de connexion électrique conformément à l'une des revendications n°1 à n°7 pour un dispositif médical implantable conformément à la revendication n°8 ou pour un stimulateur cardiaque, un défibrillateur, un neurostimulateur, un implant cochléaire, un moniteur de glucose ou une pompe de perfusion implantable conformément à la revendication n°9.
